# EUROPÄISCHE PATENTSCHRIFT

(11) **EP 1 735 034 B1**
(45) Veröffentlichungstag und Bekanntmachung des Hinweises auf die Patenterteilung: **20.04.2011**
(21) Anmeldenummer: 05715436.1
(22) Anmeldetag: 22.02.2005
(51) Int. Cl.: A61M 15/00, A61M 11/00, B65D 83/14

(54) **IMPAKTIONSDÜSE FÜR TREIBGASBETRIEBENE DOSIERAEROSOLE**
IMPACTION NOZZLE FOR A GAS-OPERATED DOSING AEROSOL
BUSE D'IMPACT DESTINEE A DES AEROSOLS DE DOSAGE FONCTIONNANT AVEC UN GAZ PROPULSEUR

(30) Priorität: 05.03.2004 DE 102004011381
(43) Veröffentlichungstag der Anmeldung: 27.12.2006
(73) Patentinhaber: BOEHRINGER INGELHEIM INTERNATIONAL GMBH, 55216 Ingelheim (DE); Boehringer Ingelheim Pharma GmbH & Co. KG, 55216 Ingelheim (DE)
(72) Erfinder: BOECK, Georg, 88471 LAUPHEIM (DE)
(74) Vertreter: Hammann, Heinz
(86) Internationale Anmeldenummer: PCT/EP2005/001803
(87) Internationale Veröffentlichungsnummer: WO 2005/087298

(56) Entgegenhaltungen:
- EP-A- 0 261 649
- WO-A1-98/23382
- WO-A1-03/097139
- GB-A- 2 324 121
- US-A- 2 941 696
- US-A- 3 367 330
- US-A- 3 383 879
- US-A- 3 406 913
- US-A- 3 730 437
- US-A- 3 920 158
- US-A- 5 662 271
- US-A1- 2003 075 623

## Beschreibung

Die vorliegende Erfindung betrifft eine neue Düse bzw. ein neues Düsensystem für treibgasbetriebene Inhalatoren für die inhalative Applikation von Aerosolformulierungen in die Lunge.

### Hintergrund der Erfindung

In treibmittelbetriebenen Inhalatoren werden die Wirkstoffe zusammen mit dem Treibmittel in patronenähnlichen Kanistern gelagert. Diese Kanister bestehen in der Regel aus einem Aluminiumbehälter, der mit einer Ventiltasse aus Aluminium verschlossen ist, in die ein Ventil eingebettet ist. Ein solcher Kanister kann dann wie eine Patrone in den Inhalator eingeführt und verbleibt dort entweder permanent oder wird nach Gebrauch gegen eine neue Patrone ausgetauscht.

Üblicherweise wird der Kanister oder Behälter aus einem Mantel gebildet, der aus Aluminium besteht, einem von Innen mit einem inerten Kunststoff ummantelten Aluminium, einem Edelstahl u.ä.. Einer der Behälter weist dabei meist vier verschiedene Zonen auf: den plan oder konkav nach innen gewölbten Boden, einen zylinderfömigen Bauchbereich, der im oberen Drittel in einen sich verjüngenden Hals übergeht und schließlich in einer Kante, z.B. Crimpkante endet, der die Öffnung des Behälters umrandet.

Typischerweise sind diese Behälter derart dimensioniert, dass sie ein Volumen von 5 bis 50 ml aufnehmen können.

Im verschlossenen Zustand ist der Behälter - meist nach dem Befüllen mit der Arzneistoff-Formulierung und dem Treibmittel mit der Ventiltasse dicht verschlossen. Als Beispiel für eine Ventiltasse wird auf die GB 2324121 verwiesen.

Im geschlossenen Zustand des Kanisters umcrimpt die Ventiltasse den Behälter an dessen Kante an der Öffnung. Meist dichtet eine Dichtung die Ventiltasse gegenüber dem Behälter ab. Die Dichtung kann dabei von ringförmiger oder scheibenförmiger Gestalt sein und besteht aus Materialien, die für die Verwendung von Arzneimittel-Formulierungen mit Fluorkohlenwasserstoffen als Treibmittel geeignet sind. Beispielsweise eignen sich hierfür Thermoplaste, Elastomere, Materialien wie Neopren, Isobutylen, Isopren, Butyl-Kautschuk, Buna-Kautschuk, Nitril-Kautschuk, Copolymere aus Ethylen und Propylen, Terpolymere aus Ethylen, Propylen und einem Dien, beispielsweise Butadien, oder fluorierte Polymere. Bevorzugtes Material sind Ethylen/Propylen-Dien-Terpolymere (EPDM).

Die Ventiltasse wird von einem Ventil durchdrungen, das auf der zum Inneren des Behälters zugewandten Seite einen Ventilstamm aufweist und nach außen hin eine Düse zum Zerstäuben der treibgashaltigen Aerosolformulierung. Das Ventil wird dabei in der zentralen Öffnung durch eine Dichtung gegenüber der Ventiltasse abgedichtet. Im einfachsten Fall ist das Ventil von zylinderartiger Gestalt. Das Fußende dieses Zylinders ragt dabei in das Innere des Behälters, das kopfseitige Ende ragt aus dem Behälter heraus. Das kopfseitige Ende trägt die Düsenöffnung. Das fußseitige Ende weist einen Einlass für die Flüssigkeit bzw. das Gas im Inneren des Behälters auf.

Derartige Ventile weisen ihrem Inneren weitere Elemente wie Federn oder Ventilkörper auf. Durch vertikale entgegen einer Feder gerichtete Bewegung in den Behälter hinein, wird das Ventil geöffnet. Eine Feder wirkt dieser Bewegung entgegen und bewirkt das automatische Verschließen des Ventils nach einer Betätigung.

Wird eine Aerosolformulierung durch einen aus dem Stand der Technik bekannten Treibgasinhalator durch Ausnutzung der adiabatischen Entspannung des Treibmittels in ein Aerosol zerstäubt wird, so weist das erzeugte Aerosol eine hohe Geschwindigkeit auf. Dies liegt daran, dass das Treibmittel einen hohen Dampfdruck aufweist und dadurch beim Öffnen des Ventils ein entsprechend hoher Druck freigesetzt wird. Diese hohen Geschwindigkeiten führen dazu, dass ein bedeutender Teil des erzeugten Aerosols bereits im Mund-Rachenraum des inhalierenden Patienten hängen bleibt und damit am beabsichtigten Wirkort Lunge nicht mehr zur Verfügung steht.

Dokument US3730437 offenbart ein Kanister mit einem Ventilsystem nach den Merkmalen des Oberbegriffs des Anspruchs 1. Aus dem Dokument US2941696 ist ein Aerosolbehälter mit zwei Ventilen die über eine Kappe miteinander verbunden sind, wobei die zwei Ventile mit zwei separaten Kammern kommunizieren.

### Beschreibung der Erfindung

Es ist eine Aufgabe der Erfindung, die aus dem Stand der Technik bekannten Nachteile von Treibgasinhalatoren zu reduzieren.

Insbesondere ist es eine Aufgabe, die Ventile von treibgasbetriebenen Inhalatoren so zu verändern, dass Aerosole mit niedrigerer Geschwindigkeit erzeugt werden.

Die vorliegende Erfindung löst diese Aufgabe dadurch, dass ein Treibgasinhalator bestückt wird mit einem Ventil bzw. Ventilsystem, durch das wenigstens zwei Wolken von Aerosolen bzw. wenigstens zwei Sprühstrahlen geschaffen wird, die sich in einem Winkel von > 0° und ≤ 180° aufeinander zu bewegen, so dass die einzelnen Aerosolpartikel zumindest partiell aufeinander treffen und dabei an kinetischer Energie verlieren. Dabei sind die Düsenöffnungen bevorzugt derart gerichtet, dass sich die geradlinigen Verlängerungen der Düsenkanäle über die Öffnung hinaus und die Längsachse der Düse in einer Ebene befinden. D.h. in allen Ausführungsformen sind die Bereiche der Kanäle die die Düsenöffnungen bilden bevorzugt gegenüber der längsseitigen Hauptachse des jeweiligen Ventils mit einem Winkel von > 0° und ≤ 90° abgewinkelt. Bevorzugt sind Winkel von > 0° und ≤ 45°. Dabei bedeutet ein Winkel von 0°, dass die Ventilöffnung mit der Längsseite des Ventils ausgerichtet ist.

Der Aerosolbehälter enthält wenigstens zwei Ventile. Bei den wenigstens zwei Ventilen sind die Kanäle im dem Bereich vor den Öffnungen bis hin zu den Öffnungen der Düsen so abgewinkelt, dass sich die austretenden Sprühstrahlen oder Aerosolwolken mit einem Winkel alpha, mit 0°< alpha ≤180° aufeinander zu bewegen.

Die wenigstens zwei Ventile sind durch ein Mittel miteinander verbunden, so dass die wenigsten zwei Ventile nur gemeinsam vertikal in das Innere des Behälters bewegt werden können und dadurch die Ventile immer zeitgleich geöffnet werden. Ein solches Mittel umfasst z.B. eine brückenartige steife und an beiden Ventilen fest verbundene Brücke oder eine gemeinsame Kappe, die den kopfseitigen Bereich der beiden Ventile überdeckt.

Eine Kappe weist dabei eine oder mehrer Öffnung(en) über den jeweiligen Düsenöffnungen der einzelnen Ventile auf.

Eine Brücke kann die beiden Ventile sowohl kopfseitig, wie auch fußseitig miteinander verbinden, d.h. auf der außerhalb des Behälters liegenden Seite oder der innerhalb des Behälters liegenden Seite.

In einer bevorzugten Ausführungsform wird die der Erfindung zugrunde liegende Aufgabe durch eine Düse gelöst, die zumindest in ihrem kopfseitigen Ende eine Mehrkanaldüse ist, deren Kanäle in dem Bereich vor der Düsenöffnung bis hin zur Düsenöffnung in einem solchen Winkel zueinander geneigt sind, dass die durch sie strömenden Aerosol-Jets stromabwärts hinter der Düse aufeinander treffen.

Die erfindungsgemäße Düse zeichnet sich dadurch aus, dass die primären Aerosolpartikel mit einer hohen kinetischen Energie erzeugt werden, so dass der Feinpartikelanteil entsprechend hoch ist. Die primären Partikel werden durch die Jet-Impaktion anschließend wieder abgebremst. Die freiwerdende Energie führt unter Umständen zu einer weiteren Verkleinerung dieser primären, treibgashaltigen Aerosolpartikel zu den Sekundär-Aerosolpartikeln.

Gemäß einer besonders bevorzugten Ausführungsform ist vorgesehen, dass die Düse eine Zweikanaldüse ist, deren beiden Kanäle jeweils im Fußbereich des Ventils, d.h. dem Ventilstamm beginnen und durch das Ventil ziehen, bis sie im Bereich der Düsenöffnungen mit einem Winkel alpha, mit 0°< alpha ≤180°, gegeneinander zu geneigt sind, so dass sich die austretenden Sprühstrahlen oder Aerosolwolken mit einem Winkel alpha, mit 0°< alpha ≤180° aufeinander zu bewegen. Der am besten geeignete Winkel wird in Abhängigkeit unter anderem von der Zusammensetzung der Treibgasformulierung und weiteren Parametern ermittelt.

Die Kanäle in einer solchen Mehrkanaldüse sind bevorzugt derart, dass sie bei gleichen Durchmessern gleiche Längen aufweisen, so dass die Zeit einer Aerosolformulierung durch die Düse immer gleich lang ist, unabhängig davon, welcher Kanal durchlaufen wird. D.h. die Wegstrecke durch die Düsen sind gleich lang, bei gleichem Durchmesser. Diese Ausgestaltung hat den Vorteil, dass keiner der wenigstens zwei Aerosolstrahlen vorzeitig aus der Düse austreten kann, ohne einen Impaktionspartner zu haben.

Andere Ausführungsformen dieser Art, weisen mehr als zwei solcher Kanäle auf.

In einer weiteren Ausführungsform, ist am fußseitigen Ende des Ventils nur ein Kanal ausgebildet, der sich innerhalb des Ventilkorpus in wenigstens zwei Kanäle aufspaltet, die den gleichen Durchlasswiderstand aufweisen. Bevorzugt sind die beiden Kanäle gleich lang und von gleichem Durchmesser. Auch hier bewegen sich die beiden Kanäle im Bereich der Düsenöffnung aufeinander zu, so dass sich die austretenden Sprühstrahlen oder Aerosolwolken mit einem Winkel alpha, mit 0°< alpha ≤180° aufeinander zu bewegen.

Andere Ausführungsformen dieser Art, weisen im Verästelungsbereich mehr als zwei solcher kopfseitigen Kanäle auf.

### Figuren

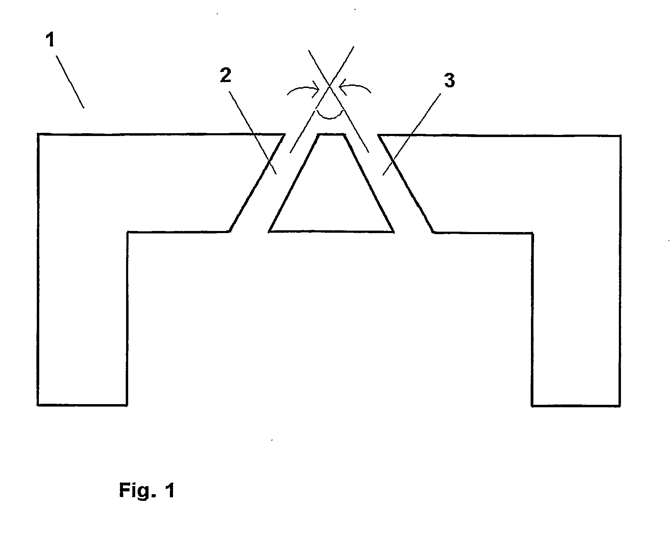

Fig. 1 zeigt schematisch eine Mehrkanaldüse 1 in Ausbildung einer Zweikanaldüse mit zwei Kanälen 2 und 3. Diese Mehrkanaldüse kann im Kopfbereich eines Ventils als Sprühkopf ausgebildet sein. Die Kanäle 2 und 3 sind zueinander in einem Winkel alpha geneigt. Zur Darstellung des Winkels ist die virtuelle Längsachse jedes Kanals dargestellt, deren Schnittpunkt dem Impaktionspunkt der Aerosol-Jets/Wolken entspricht. Der Winkel wird durch die zwei gebogenen Pfeile am Impaktionspunkt und die die beiden Längsachsen verbindende Rundung angezeigt. Hierbei kann der Winkel alpha zwischen > 0° und 180° liegen. Die Neigung führt nicht nur dazu, dass ein aus dem Kanal 1 mit einem Aerosol-Jet/Wolke aus dem Kanal 2 impaktiert, sondern auch, dass sich die Aerosolteilchen gegenseitig abbremsen. In der Ausbildung nach Fig. 1 werden beide Kanäle 2 und 3 einheitlich mit der Treibgasformulierung beschickt.

Die Aerosol-Jets können - wie ausgeführt - in jedem beliebigen Winkel von > 0° bis 180°, in jedem beliebigen Abstand von der Oberfläche der Düsenöffnung und mit jeder beliebigen Geschwindigkeit aufeinander prallen. Das Verhältnis der Parameter entscheidet über den Grad der Dispergierung und über die Geschwindigkeit der entstehenden Sekundär-Aerosolwolke.

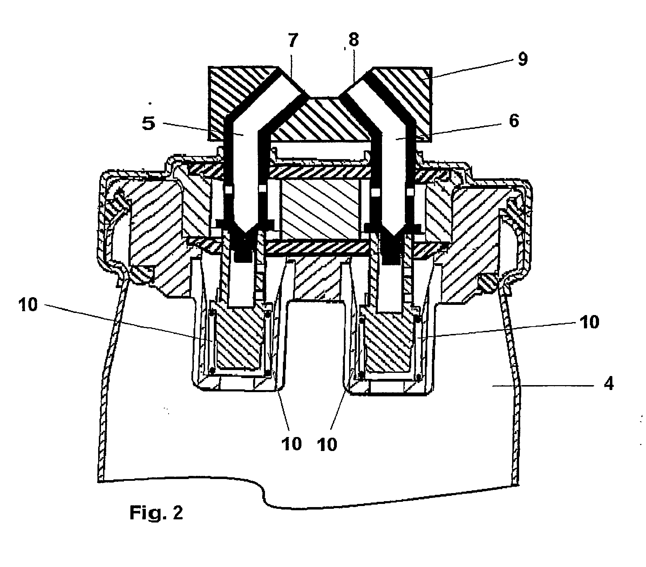

Fig. 2 zeigt eine Ausführungsform eines Dosieraerosolbehälters 4 mit zwei Ventilen 5 und 6, der Öffnungen 7 und 8 erfindungsgemäß gegeneinander geneigt sind. Die beiden Ventile 5 und 6 sind mit einer gemeinsamen Kappe 9 bestückt, die wenigstens eine Öffnung vor den Ventilöffnungen 7 und 8 aufweist, so dass die beiden Ventile 5 und 6 nur gemeinsam bewegt werden können. Die Ventile können senkrecht gegen die Federn 10 in den Behälter hinein bewegt werden. In der Zeichnung sind die beiden Ventilstämme so dargestellt, dass sie vom ihrem Fußende bis kurz oberhalb der Ventiltasse parallel zur Längsachse des Behälters (Senkrechte von oben nach unten) verlaufen und dann oberhalb der Ventiltasse aufeinander zu abknicken. In anderen Ausführungsformen ist der Ventilstamm gerade und nur die zu den Öffnungen führenden Kanäle 7 und 8 sind entsprechend abgewinkelt.

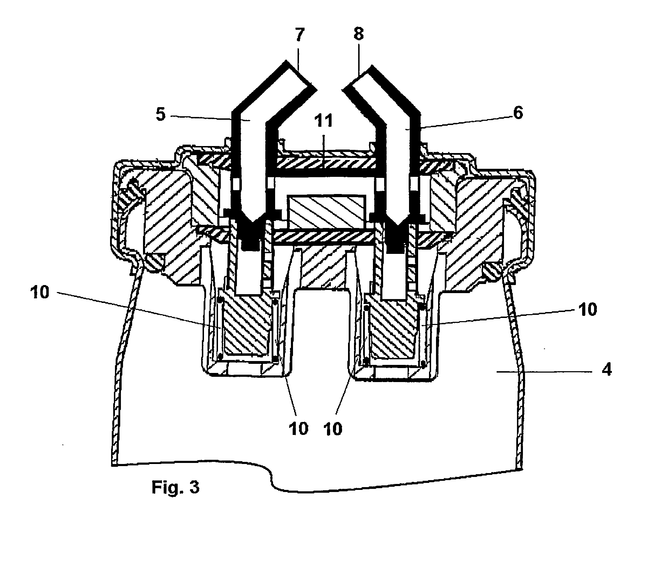

Fig. 3 zeigt eine Ausführungsform eines Dosieraerosolbehälters 4 mit zwei Ventilen 5 und 6, der Öffnungen 7 und 8 erfindungsgemäß gegeneinander geneigt sind. Die beiden Ventile 5 und 6 sind mit einer gemeinsamen Brücke 11 bestückt, die die beiden Ventile fest und steif miteinander verbindet, so dass die beiden Ventile 5 und 6 nur gemeinsam bewegt werden können. Auch für diese Ausführungsform gilt bezüglich der äußeren Ventilgestaltung, dass nicht zwangsläufig die Ventilstämme oberhalb der Ventiltasse abgewinkelt sein müssen, sondern auch nur die Kanäle im Inneren der Ventiltasse abgewinkelt sein können (s. a. Beschreibung Fig. 2).

## Patentansprüche

1. Kanister für treibmittelbetriebene Dosierareosole zur inhalativen Applikation einer Aeroselformulierung bestehend aus einem Behälter (4) und einer Ventiltasse mit wenigstens einem eingebettetem, die Ventiltasse durchdringenden Ventil oder Ventilsystem, wobei
- das fußseitige Ende des Ventils oder Ventilsystems in den Behälter (4) hineinragt und das kopfseitige Ende außerhalb des Behälters (4) liegt und
- das Ventil oder Ventilsystem einen Kanal oder mehrere Kanäle (2,3) zum Durchleiten einer Treibgasformulierung aus dem Inneren des Behälters (4) nach Außen aufweist und
- das Ventil oder Ventilsystem wenigstens zwei äußere, düsenartige Ventilöffnungen (7,8) aufweist, die mit dem wenigstens einen Kanal (2,3) verbunden sind und die so ausgerichtet sind, dass die daraus austretenden Flüssigkeitsstrahlen, Aerosolstrahlen oder Aerosolwolken mit einem Winkel von > 0° und ≤ 180° aufeinander zu gerichtet sind so dass die einzelnen Aerosolpartikel zumindest partiell aufeinander treffen und dabei an kinetischer Energie verlieren und
- das Ventil oder Ventilsystem partiell senkrecht durch die Ventiltasse beweglich ist, und **dadurch** geöffnet wird,
**dadurch gekennzeichnet, dass** es sich bei dem Ventil oder Ventilsystem um ein Ventilsystem aus wenigstens zwei zylinderartigen Ventilen (5, 6) handelt, deren Längsachsen die Ventiltasse senkrecht durchdringen und die durch eine steife Brücke (11) fest miteinander verbunden sind, so dass die beiden Ventile (5, 6) nur gemeinsam bewegt werden können

2. Kanister nach Anspruch 1, **dadurch gekennzeichnet, dass** das Ventil oder Ventilssystem wenigstens zwei nicht miteinander verbundene Kanäle (2, 3) aufweist, die in jeweils einer Düsenöffnung (7,8) enden.

3. Kanister nach Anspruch 1, **dadurch gekennzeichnet, dass** das Ventil oder Ventilssystem zwei nicht miteinander verbundene Kanäle aufweist (2, 3), die in jeweils einer Düsenöffnung (7, 8) enden.

4. Kanister nach Anspruch 1, **dadurch gekennzeichnet, dass** am fußseitigen Ende des Ventils oder Ventilssystems ein einziger Kanal ausgebildet ist, der sich im Inneren des Ventils oder Ventilssystems in wenigstens zwei Äste aufteilt, die jeweils zu einer Düsenöffnung führen.

5. Kanister nach Anspruch 4, **dadurch gekennzeichnet, dass** die wenigstens zwei Äste gleiche Längen und Durchmesser aufweisen.

6. Kanister nach einem der vorhergehenden Ansprüche, **dadurch gekennzeichnet, dass** die Senkrechtbewegung des Ventils oder Ventilsystems in den Behälter gegen eine Feder gerichtet ist

7. Kanister nach einem der vorhergehenden Ansprüche, **dadurch gekennzeichnet, dass** die durch die Düsenöffnungen verlängerten Geraden und die Senkrechte zur Ventiltasse in einer Ebene liegen.

8. Kanister nach einem der vorhergehenden Ansprüche, **dadurch gekennzeichnet, dass** die durch die die Düsenöffnungen verlängerten Geraden gebildete Ebene und die Senkrechte zur Ventiltasse windschief zueinander stehen.

9. Kanister nach Anspruch 8, **dadurch gekennzeichnet, dass** die Senkrechte zur Ebene und die Senkrechte zur Ventiltasse eine zweite Ebenen aufspannen.

10. Kanister nach Anspruch 8, **dadurch gekennzeichnet, dass** die Senkrechte zur Ebene und die Senkrechte zur Ventiltasse windschief zueinander stehen.

11. Kanister nach Anspruch 1, **dadurch gekennzeichnet, dass** die Brücke (11) eine Kappe (9) ist, die die beiden kopfseitigen Enden der Ventile (5, 6) überdeckt, die wenigstens eine Öffnung enthält, die Ventilöffnungen nach Außen frei gibt.

12. Kanister nach Anspruch 1, **dadurch gekennzeichnet, dass** die Brücke (11) eine fest Verbindung außerhalb des Behälter (4) ist.

13. Kanister nach Anspruch 1, **dadurch gekennzeichnet, dass** die Brücke (11) eine fest Verbindung innerhalb des Behälter (4) ist.

## Claims

1. Canister for propellant driven metered dose aerosols for the inhalative administration of an aerosol formulation, consisting of a container (4) and a valve cup with at least one valve or valve system embedded therein and penetrating through the valve cup, where
- the base end of the valve or valve system projects into the container (4) and the head end is located outside the container (4) and
- the valve or valve system has one channel or a plurality of channels (2, 3) for conveying a propellant gas formulation from the interior of the container (4) to the outside and
- the valve or valve system comprises at least two external, nozzle-like valve openings (7, 8) which are connected to the minimum of one channel (2, 3) and which are aligned so that the fluid jets, aerosol jets or aerosol clouds emerging from them are directed towards one another at an angle of >0° and ≤180° so that the individual aerosol particles at least partially collide with one another and in doing so lose kinetic energy and
- the valve or valve system is partially perpendicularly moveable through the valve cup and is thereby opened,
**characterised in that** the valve or valve system is a valve system consisting of at least two cylindrical valves (5, 6), the longitudinal axes of which pass perpendicularly through the valve cup and which are fixedly joined together by a rigid bridge (11), so that the two valves (5, 6) can only be moved jointly.

2. Canister according to claim 1, **characterised in that** the valve or valve system comprises at least two channels (2, 3) which are not connected to one another, each of which terminates in a nozzle opening (7, 8).

3. Canister according to claim 1, **characterised in that** the valve or valve system comprises two channels (2, 3) not connected to one another each of which terminates in a nozzle opening (7, 8).

4. Canister according to claim 1, **characterised in that** at the base end of the valve or valve system is formed a single channel which divides inside the valve or valve system into at least two branches, each of which leads to a nozzle opening.

5. Canister according to claim 4, **characterised in that** the minimum of two branches have the same length and diameter.

6. Canister according to one of the preceding claims, **characterised in that** the perpendicular movement of the valve or valve system into the container is directed counter to a spring.

7. Canister according to one of the preceding claims, **characterised in that** the straight lines extended through the nozzle openings and the perpendicular to the valve cup are situated in one plane.

8. Canister according to one of the preceding claims, **characterised in that** the plane formed by the straight lines extended through the nozzle openings and the perpendicular to the valve cup are at an angle to one another.

9. Canister according to claim 8, **characterised in that** the perpendicular to the plane and the perpendicular to the valve cup span a second plane.

10. Canister according to claim 8, **characterised in that** the perpendicular to the plane and the perpendicular to the valve cup are at an angle to one another.

11. Canister according to claim 1, **characterised in that** the bridge (11) is a cap (9) which covers the two head ends of the valves (5, 6) and which contains at least one opening which leaves the valve openings open to the outside.

12. Canister according to claim 1, **characterised in that** the bridge (11) is a fixed connection outside the container (4).

13. Canister according to claim 1, **characterised in that** the bridge (11) is a fixed connection inside the container (4).

## Revendications

1. Récipient pour des aérosols doseurs, actionnés par un moyen de propulsion, pour l'administration par inhalation d'une formulation d'un aérosol, formé par un récipient (4) et un logement de valve comportant au moins une valve ou système de valves encastré, traversant le logement de valve, sachant que
- l'extrémité inférieure de la valve ou du système de valves s'engage dans le récipient (4) et l'extrémité supérieure se situe en dehors du récipient (4), et
- la valve ou le système de valves comporte un conduit ou plusieurs conduits (2, 3) pour le passage d'une formulation de gaz propulseur depuis l'intérieur du récipient (4) vers l'extérieur, et
- la valve ou le système de valves comporte au moins deux ouvertures (7, 8) extérieures, du type buse, qui communiquent avec ledit au moins un conduit (2, 3) et qui sont orientées de telle sorte que les jets de liquide, jets d'aérosol ou nuages d'aérosol qui en sortent sont dirigés les uns vers les autres selon un angle > 0° et ≤ 180°, de telle sorte que les différentes particules d'aérosol se rencontrent au moins en partie et, à cette occasion, perdent de l'énergie cinétique, et
- la valve ou le système de valves est mobile en partie verticalement à travers le logement de valve et est ouvert de ce fait,
**caractérisé en ce que** la valve ou le système de valves est un système de valves formé par au moins deux valves cylindriques (5, 6), dont les axes longitudinaux passent verticalement à travers le logement de valve et lesquelles sont reliées de manière fixe l'une à l'autre par un pont (11) rigide, de telle sorte que les deux vannes (5, 6) peuvent être déplacées seulement conjointement.

2. Récipient selon la revendication 1, **caractérisé en ce que** la valve ou le système de valves comporte au moins deux conduits (2, 3) non reliés entre eux, qui se terminent chacun dans une ouverture de buse (7, 8).

3. Récipient selon la revendication 1, **caractérisé en ce que** la valve ou le système de valves comporte deux conduits (2, 3) non reliés entre eux, qui se terminent chacun dans une ouverture de buse (7, 8).

4. Récipient selon la revendication 1, **caractérisé en ce qu'**au niveau de l'extrémité inférieure de la valve ou du système de valves est réalisé un seul conduit qui, à l'intérieur de la valve ou du système de valves, se divise en au moins deux branches, qui conduisent chacune vers une ouverture de buse.

5. Récipient selon la revendication 4, **caractérisé en ce que** lesdites au moins deux branches ont la même longueur et le même diamètre.

6. Récipient selon l'une quelconque des revendications précédentes, **caractérisé en ce que** le mouvement vertical de la valve ou du système de valves dans le récipient est dirigé à l'encontre d'un ressort.

7. Récipient selon l'une quelconque des revendications précédentes, **caractérisé en ce que** les droites prolongées par les ouvertures de buse et les verticales au logement de valve sont situées dans un plan.

8. Récipient selon l'une quelconque des revendications précédentes, **caractérisé en ce que** le plan formé par les droites prolongées par les ouvertures de buse et les verticales au logement de valve sont disposées de manière inclinée les unes par rapport aux autres.

9. Récipient selon la revendication 8, **caractérisé en ce que** les verticales au plan et les verticales au logement de valve déploient un deuxième plan.

10. Récipient selon la revendication 8, **caractérisé en ce que** les verticales au plan et les verticales au logement de valve sont disposées de manière inclinée les unes par rapport aux autres.

11. Récipient selon la revendication 1, **caractérisé en ce que** le pont (11) est un capuchon (9), qui couvre les deux extrémités frontales des valves (5, 6), qui comporte au moins une ouverture, qui libère les ouvertures de valve vers l'extérieur.

12. Récipient selon la revendication 1, **caractérisé en ce que** le pont (11) est une liaison fixe à l'extérieur du récipient (4).

13. Récipient selon la revendication 1, **caractérisé en ce que** le pont (11) est une liaison fixe à l'intérieur du récipient (4).
